Europäisches Patentamt

**European Patent Office**

Office européen des brevets

(11) Numéro de publication: **0 284 481**
**B1**

(19)

(12) **FASCICULE DE BREVET EUROPEEN**

(45) Date de publication du fascicule du brevet:
**28.11.90**

(21) Numéro de dépôt: **88400539.8**

(22) Date de dépôt: **08.03.88**

(51) Int. Cl.⁵: **C07C 233/09**

(54) **Acide acrylamidoglycolique cristallisé pur anhydre et son procédé de préparation.**

(30) Priorité: **16.03.87 FR 8703545**

(43) Date de publication de la demande:
**28.09.88 Bulletin 88/39**

(45) Mention de la délivrance du brevet:
**28.11.90 Bulletin 90/48**

(84) Etats contractants désignés:
**AT BE CH DE ES FR GB GR IT LI LU NL SE**

(56) Documents cités:
**FR-A- 1 411 715**
**US-A- 4 443 623**

**BULLETIN DE LA SOCIETE CHIMIQUE DE FRANCE,**
**partie II, mai-juin 1978, pages 248-254; A.**
**SCHOUTEETEN et al.: "Les N-acylhémiaminals de**
**l'acide glyoxylique et leur utilisation en synthèse"**

(73) Titulaire: **SOCIETE FRANCAISE HOECHST Société**
**anonyme dite:, 3, avenue du Général de Gaulle,**
**F-92800 Puteaux(FR)**

(72) Inventeur: **Sidot, Christian, Résidence des Fleurs**
**Avenue Foch, F-95460 Ezanville(FR)**
Inventeur: **Christidis, Yani, 55 Boulevard de Villette,**
**F-75019 Paris(FR)**

(74) Mandataire: **Rinuy, Santarelli et al, 14, avenue de la**
**Grande Armée, F-75017 Paris(FR)**

## Description

La présente invention concerne l'acide acrylamidoglycolique cristallisé pur anhydre et son procédé de préparation.

Le brevet français N° 1.411.715 décrit l'acide acrylamidoglycolique cristallisé avec une molécule d'eau. Cet acide, présentant un point de fusion de 95°C, est insoluble dans les solvants chlorés et les solvants non polaires, il est également peu soluble dans les monomères acryliques.

Le procédé généralement utilisé pour sa préparation consiste à faire réagir de l'acide glyoxylique avec l'acrylamide en solution aqueuse à pH alcalin en présence d'un catalyseur comme, par exemple, du carbonate de sodium.

On recherchait depuis longtemps de l'acide acrylamidoglycolique anhydre cristallisé mais tous les essais de désolvatation de l'acide acrylamidoglycolique cristallisé avec une molécule d'eau se heurtaient à l'instabilité de cet acide.

De ce fait, on attendait vainement de l'acide acrylamidoglycolique cristallisé anhydre afin de pouvoir notamment l'incorporer aisément dans des copolymères vinyliques ou acryliques en solution ou en dispersion non aqueuses. En effet, cet acide est un monomère réticulant qui apporte aux copolymères qui le contiennent des fonctions acides sous une forme originale.

Or la Demanderesse a découvert à présent qu'il est possible d'obtenir avantageusement l'acide acrylamidoglycolique cristallisé pur anhydre avec un bon rendement par réaction de l'acide glyoxylique avec l'acrylamide. L'acide acrylamidoglycolique cristallisé pur anhydre est obtenu directement, sans qu'il soit nécessaire de procéder à sa purification par des traitements subséquents. Il présente une excellente stabilité au stockage, il n'est pas hygroscopique et il est parfaitement incolore.

L'invention a pour objet l'acide acrylamidoglycolique cristallisé pur anhydre.

L'invention a également pour objet un procédé pour produire de l'acide acrylamidoglycolique cristallisé pur anhydre, par réaction de l'acrylamide avec de l'acide glyoxylique en solution aqueuse de concentration égale ou supérieure à 60 % et à une température comprise entre 35°C et 80°C, procédé caractérisé par le fait que la réaction est réalisée à un pH inférieur à 7, en l'absence de catalyseur de quelque nature que ce soit.

Il est surprenant que la condensation entre l'acrylamide et l'acide glyoxylique puisse être effectuée à un pH inférieur à 7, car il est généralement admis que ce type de réaction de N-méthylolation des groupements amides exige un pH alcalin.

Avantageusement, le procédé décrit ci-dessus est mis en oeuvre au sein d'un solvant organique compatible partiellement soluble dans l'eau tel que l'acétate d'éthyle, l'acétate de butyle, l'acétone, la méthyl-éthylcétone, la méthylisobutylcétone, le tétrahydrofuranne, l'éther diméthylique du diéthylèneglycol.

L'exemple suivant illustre l'invention sans toutefois la limiter.

### Exemple

On introduit lentement en 5 minutes environ 213 g (3 moles) d'acrylamide dans une solution agitée, constituée par :
- 277 g d'acétate d'éthyle,
- 277 g d'une solution aqueuse d'acide glyoxylique à 80 % en poids, soit 3 moles d'acide glyoxylique,
- 0,24 g d'éther monométhylique de l'hydroquinone.

La dissolution de l'acrylamide est endothermique. La solution obtenue est ensuite chauffée à 50°C environ pendant 210 minutes puis elle est refroidie à la température ambiante. L'acide acrylamidoglycolique anhydre cristallise spontanément. On l'essore puis on le lave par empâtage avec de l'acétate d'éthyle glacé et enfin on le sèche sous vide à 35°C à poids constant.

On obtient ainsi 340 g (2,34 moles) d'acide acrylamidoglycolique cristallisé pur anhydre, incolore, présentant un point de fusion instantané de 125 ± 2°C avec décomposition, soit un rendement de 78 % de la théorie.

Microanalyse

|  |  | C % | H % | N % | O % |
|---|---|---|---|---|---|
| $C_5H_7NO_4$ | calculés | 41,37 | 4,86 | 9,65 | 44,12 |
| PM = 145,11 | trouvés | 41,6 | 4,9 | 9,7 |  |
| Dosage acidimétrique: |  | 100% |  |  |  |
| Dosage des doubles liaisons: |  | 100% |  |  |  |

Cet acide présente une solubilité dans l'eau de 12 %, dans l'acétate d'éthyle de 0,25 %, dans l'acétone de 2,5 %, dans l'acrylate de butyle de 0,25 %.

A la connaissance de la Demanderesse, ce produit n'est pas décrit dans la littérature.

Il va du reste de soi que la présente invention n'a été décrite qu'à titre purement explicatif et nullement limitatif et que toute modification, notamment au niveau des équivalences, pourra y être apportée sans sortir de son cadre.

## Revendications

1. Acide acrylamidoglycolique cristallisé pur anhydre.

2. Procédé pour produire de l'acide acrylamidoglycolique cristallisé pur anhydre par réaction de l'acrylamide avec de l'acide glyoxylique en solution aqueuse de concentration égale ou supérieure à 60 % à une température comprise entre 30°C et 80°C, caractérisé par le fait que cette réaction est réalisée à un pH inférieur à 7, en l'absence de catalyseur de quelque nature que ce soit.

3. Procédé selon la revendication 2, caractérisé par le fait qu'il est effectué en présence d'un solvant organique choisi dans le groupe constitué par l'acétate d'éthyle, l'acétate de butyle, l'acétone, la méthyl-éthylcétone, la méthylisobutylcétone, le tétrahydrofuranne, l'éther diméthylique du diéthylèneglycol.

## Claims

1. Pure anhydrous crystallized acrylamido glycolic acid.

2. A process for the preparation of pure anhydrous crystallized acrylamido glycolic acid by reacting acrylamide with glyoxylic acid in aqueous solution of a concentration equal to or greater than 60% at a temperature of between 30°C and 80°C, characterized in that the reaction is performed with a pH lower than 7 in the absence of any catalyst whatsoever.

3. A process according to claim 2, characterized in that it is performed in the presence of an organic solvent selected from the group formed by ethyl acetate, butyl acetate, acetone, methylethyl ketone, methyl-isobutyl ketone, tetrahydrofuran, diethylene glycol dimethyl ether.

## Patentansprüche

1. Wasserfreie reine kristallisierte Acrylamidoglykolsäure.

2. Verfahren zur Herstellung der wasserfreien reinen kristallisierten Acrylamidoglykolsäure durch Umsetzung von Acrylamid mit Glyoxylsäure in wässeriger Lösung bei einer Konzentration von 60% oder darüber bei einer Temperatur von 30 bis 80°C, dadurch gekennzeichnet, daß diese Umsetzung bei einem pH von weniger als 7 in Abwesenheit eines Katalysators, von welcher Natur auch immer, durchgeführt wird.

3. Verfahren nach Anspruch 2, dadurch gekennzeichnet, daß es in Anwesenheit eines organischen Lösungsmittels ausgewählt aus der Gruppe bestehend aus Äthylacetat, Butylacetat, Aceton, Methyläthylketon, Methylisobutylketon, Tetrahydrofuran und Diäthylenglykoldimethyläther, durchgeführt wird.